(19) Europäisches Patentamt
European Patent Office
Office européen des brevets

(11) Veröffentlichungsnummer: **0 543 245 A1**

# (12) EUROPÄISCHE PATENTANMELDUNG

(21) Anmeldenummer: **92119128.4**

(22) Anmeldetag: **09.11.92**

(51) Int. Cl.[5]: **C07D 487/04**, A61K 31/495, A61K 31/53, //(C07D487/04, 249:00,241:00),(C07D487/04, 253:00,249:00)

(30) Priorität: **19.11.91 CH 3374/91**
**28.08.92 CH 2665/92**

(43) Veröffentlichungstag der Anmeldung:
**26.05.93 Patentblatt 93/21**

(84) Benannte Vertragsstaaten:
**AT BE CH DE DK ES FR GB GR IE IT LI LU MC NL PT SE**

(71) Anmelder: **F. HOFFMANN-LA ROCHE AG**
**Grenzacherstrasse 124**
**CH-4002 Basel(CH)**

(72) Erfinder: **Stadler, Heinz**
**Waldhofstrasse 37**
**CH-4310 Rheinfelden(CH)**
Erfinder: **Vieira, Eric**
**Frobenstrasse 57**
**CH-4053 Basel(CH)**
Erfinder: **Wostl, Wolfgang**
**Im Strick 2**
**W-7889 Grenzach-Wyhlen(DE)**

(74) Vertreter: **Kellenberger, Marcus, Dr. et al**
**Grenzacherstrasse 124 Postfach 3255**
**CH-4002 Basel (CH)**

## (54) Aminosäurederivate mit reninhemmender Aktivität.

(57) Die Verbindungen der Formel

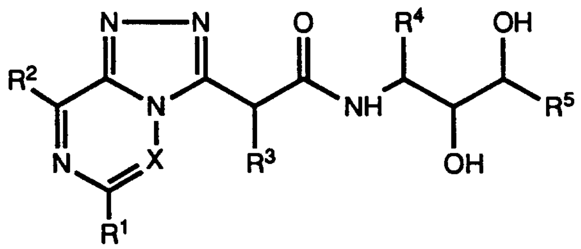

I

worin X, $R^1$, $R^2$, $R^3$, $R^4$ und $R^5$ die in Anspruch 1 angegebene Bedeutung besitzen, in Form von optisch reinen Diastereomeren, Diastereomerengemischen, diastereomeren Racematen oder Gemischen von diastereomeren Racematen sowie pharmazeutisch verwendbare Salze davon hemmen die Wirkung des natürlichen Enzyms Renin und können demnach in Form pharmazeutischer Präparate bei der Bekämpfung bzw. Verhütung von Bluthochdruck und Herzinsuffizienz verwendet werden. Sie können durch Umsetzen eines Aminodiols der Formel

EP 0 543 245 A1

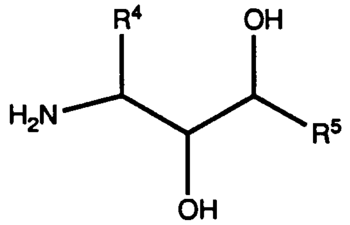

II

mit einer entsprechenden Säure oder einem aktivierten Derivat davon hergestellt werden.

Die vorliegende Erfindung betrifft Aminosäurederivate. Im speziellen betrifft sie Aminosäurederivate der allgemeinen Formel

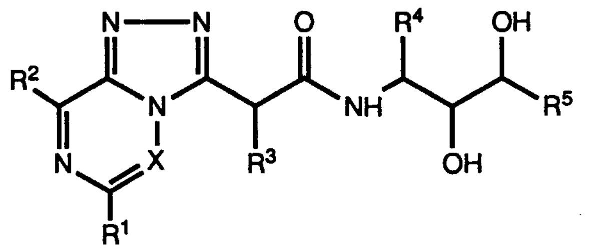

I

worin X ein Stickstoffatom oder –CH–, $R^1$ Phenyl, Pyridyl oder Isochinolinyl, $R^2$ Cycloalkyl, Cycloalkylalkyl, Alkylthioalkyl, Alkylsulfonylalkyl, Alkenyl oder, falls X ein Stickstoffatom und/oder $R^1$ Isochinolinyl und/oder $R^3$ Alkenyl bedeuten, zusätzlich Alkyl, $R^3$ Wasserstoff, Alkyl, Alkenyl, Imidazolylmethyl, Pyridylmethyl, Thiazolylmethyl oder Benzyl, $R^4$ Cyclohexylmethyl oder Benzyl und $R^5$ Cycloalkyl, Alkyl oder Heterocyclylalkyl bedeuten,
in Form von optisch reinen Diastereomeren, Diastereomerengemischen, diastereomeren Racematen oder Gemischen von diastereomeren Racematen sowie pharmazeutisch verwendbare Salze dieser Verbindungen.

Diese Verbindungen sind neu und zeichnen sich durch wertvolle pharmakodynamische Eigenschaften aus.

Gegenstand der vorliegenden Erfindung sind die Verbindungen der Formel I und ihre pharmazeutisch verwendbaren Salze als solche und zur Anwendung als therapeutische Wirkstoffe, die Herstellung dieser Verbindungen, ferner diese enthaltende Arzneimittel und die Herstellung solcher Arzneimittel, sowie die Verwendung von Verbindungen der Formel I und ihrer pharmazeutisch verwendbaren Salze bei der Bekämpfung bzw. Verhütung von Krankheiten, bzw. bei der Verbesserung der Gesundheit, insbesondere bei der Bekämpfung bzw. Verhütung von Bluthochdruck und Herzinsuffizienz.

Der in der vorliegenden Beschreibung verwendete Ausdruck "Alkyl" – allein oder in Kombination – bedeutet geradkettige und verzweigte, gesättigte Kohlenwasserstoffreste mit 1–8, vorzugsweise 1–4, Kohlenstoffatomen, wie Methyl, Aethyl, n-Propyl, Isopropyl, n-Butyl, Isobutyl, sec.-Butyl, t-Butyl, Pentyl, Hexyl und dergleichen. Der Ausdruck "Cycloalkyl" bedeutet gesättigte, cyclische Kohlenwasserstoffreste mit 3–8, vorzugsweise 3–6, Kohlenstoffatomen, wie Cyclopropyl, Cyclobutyl, Cyclopentyl, Cyclohexyl und dergleichen. Der Ausdruck "Alkenyl" betrifft geradkettige und verzweigte, ungesättigte Kohlenwasserstoffreste mit 2–8, vorzugsweise 2–4, Kohlenstoffatomen, wie Vinyl, Allyl, 2-Butenyl, 3-Butenyl und dergleichen. Der Ausdruck "Heterocyclyl" bedeutet einen gesättigten 5- oder 6-gliedrigen Heterocyclus mit mindestens einem Stickstoffatom und gegebenenfalls einem zusätzlichen Sauerstoff-, Stickstoff- oder Schwefelatom als Ringglieder, welcher über ein Stickstoffatom an die –CH(OH)-Gruppe gebunden ist und in welchem das zusätzliche Stickstoffatom alkyliert sein kann, wie Morpholinyl, Piperidinyl, Piperazinyl, Thiomorpholinyl, S-Oxothiomorpholinyl, S,S-Dioxothiomorpholinyl, Pyrrolidinyl, Thiazolidinyl, Imidazolidinyl, Oxazolidinyl, N-Methylpiperidinyl und dergleichen.

Der Ausdruck "pharmazeutisch verwendbare Salze" umfasst Salze mit anorganischen oder organischen Säuren, wie Salzsäure, Bromwasserstoffsäure, Salpetersäure, Schwefelsäure, Phosphorsäure, Zitronensäure, Ameisensäure, Maleinsäure, Essigsäure, Bernsteinsäure, Weinsäure, Methansulfonsäure, p-Toluolsulfonsäure und dergleichen. Solche Salze können im Hinblick auf den Stand der Technik und unter Berücksichtigung der Natur der in ein Salz zu überführenden Verbindung durch jeden Fachmann ohne weiteres hergestellt werden.

Die Verbindungen der Formel I besitzen mindestens drei asymmetrische Kohlenstoffatome und liegen deshalb in Form von optisch reinen Diastereomeren, Diastereomerengemischen, diastereomeren Racematen oder Gemischen von diastereomeren Racematen vor. Die vorliegende Erfindung umfasst alle Formen. Diastereomerengemische, diastereomere Racemate oder Gemische von diastereomeren Racematen können nach üblichen Methoden aufgetrennt werden, z.B. durch Säulenchromatographie, Dünnschichtchromatographie, HPLC und dergleichen.

Eine besondere Gruppe von Verbindungen der Formel I sind solche, worin $R^2$ Cycloalkyl, Cycloalkylalkyl, Alkylthioalkyl, Alkylsulfonylalkyl, Alkenyl oder, falls X ein Stickstoffatom und/oder $R^1$ Isochinolinyl bedeuten, zusätzlich Alkyl bedeutet.

Bevorzugt sind solche Verbindungen der Formel I, worin X –CH– bedeutet. Bevorzugt sind auch Verbindungen der Formel I, worin $R^1$ Pyridyl, besonders bevorzugt 3-Pyridyl, bedeutet. $R^2$ bedeutet

vorzugsweise Cycloalkyl oder Cycloalkylalkyl, besonders bevorzugt Cycloalkyl, ganz besonders bevorzugt Cyclopropyl. Die bevorzugte Bedeutung von $R^3$ ist Imidazolylmethyl, Pyridylmethyl oder Thiazolylmethyl, besonders bevorzugt Pyridylmethyl oder Thiazolylmethyl, ganz besonders bevorzugt 4-Thiazolylmethyl. Bevorzugt ist die Bedeutung Cyclohexylmethyl für $R^4$, $R^5$ bedeutet vorzugsweise Cycloalkyl oder Alkyl, besonders bevorzugt Cycloalkyl, ganz besonders bevorzugt Cyclopropyl.

Aus dem obigen folgt, dass solche Verbindungen der Formel I ganz besonders bevorzugt sind, worin X -CH-, $R^1$ 3-Pyridyl, $R^2$ Cyclopropyl, $R^3$ 4-Thiazolylmethyl, $R^4$ Cyclohexylmethyl und $R^5$ Cyclopropyl bedeuten.

Ganz speziell bevorzugte Verbindungen der Formel I sind:

(R oder S)-N-[(1S,2R,3S)-1-(Cyclohexylmethyl)-3-cyclopropyl-2,3-dihydroxypropyl]-8-cyclopropyl-6-(3-pyridyl)-α-(3-pyridylmethyl)-s-triazolo-[4,3-a]pyrazin-3-acetamid,

N-[(1S,2R,3S)-1-(Cyclohexylmethyl)-3-cyclopropyl-2,3-dihydroxypropyl]-8-cyclopropyl-6-(3-pyridyl)-s-triazolo[4,3-a]pyrazin-3-acetamid,

(R oder S)-N-[(1S,2R,3S)-1-(Cyclohexylmethyl)-3-cyclopropyl-2,3-dihydroxypropyl]-8-cyclopropyl-6-(3-pyridyl)-α-(4-thiazolylmethyl)-s-triazolo[4,3-a]pyrazin-3-acetamid,

(R,S)-N-[(1S,2R,3S)-1-Benzyl-3-cyclopropyl-2,3-dihydroxypropyl]-8-cyclopropyl-6-(3-pyridyl)-α-(3-pyridylmethyl)-s-triazolo[4,3-a]pyrazin-3-acetamid,

(R oder S)-N-[(1S,2R,3S)-1-(Cyclohexylmethyl)-3-cyclopropyl-2,3-dihydroxypropyl]-8-(cyclopropylmethyl)-6-(3-pyridyl)-α-(3-pyridylmethyl)-s-triazolo[4,3-a]pyrazin-3-acetamid,

(R oder S)-N-[(1S,2R,3S)-1-(Cyclohexylmethyl)-3-cyclopropyl-2,3-dihydroxypropyl]-8-(cyclopropylmethyl)-6-(3-pyridyl)-α-(4-thiazolylmethyl)-s-triazolo[4,3-a]pyrazin-3-acetamid und

(R,S)-α-Allyl-N-[(1S,2R,3S)-1-(cyclohexylmethyl)-3-cyclopropyl-2,3-dihydroxypropyl]-8-propyl-6-(3-pyridyl)-s-triazolo[4,3-a]pyrazin-3-acetamid.

Weitere speziell bevorzugte Verbindungen der Formel I sind:

N-[(1S,2R,3S)-1-(Cyclohexylmethyl)-3-cyclopropyl-2,3-dihydroxypropyl]-6-(4-isoctunolinyl)-8-propyl-s-triazolo[4,3-a]pyrazin-3-acetamid,

(S oder R)-N-[(1S,2R,3S)-1-(Cyclohexylmethyl)-3-cyclopropyl-2,3-dihydroxypropyl]-6-(4-isochinolinyl)-8-propyl-α-(3-pyridylmethyl)-s-triazolo-[4,3-a]pyrazin-3-acetamid und

(R oder S)-N-[(1S,2R,3S)-1-(Cyclohexylmethyl)-3-cyclopropyl-2,3-dihydroxypropyl]-6-(4-isochinolinyl)-8-propyl-α-(3-pyridylmethyl)-s-triazolo-[4,3-a]pyrazin-3-acetamid.

Weitere Beispiele bevorzugter Verbindungen der Formel I sind:

N-[(1S,2R,3S)-1-(Cyclohexylmethyl)-3-cyclopropyl-2,3-dihydroxypropyl]-8-propyl-6-(3-pyridyl)-s-triazolo[3,4-f][1,2,4]triazin-3-acetamid,

(R oder S)-N-[(1S,2R,3S)-1-(Cyclohexylmethyl)-3-cyclopropyl-2,3-dihydroxypropyl]-8-propyl-6-(3-pyridyl)-α-(3-pyridylmethyl)-s-triazolo[3,4-f]-[1,2,4]triazin-3-acetamid,

(S oder R)-N-[(1S,2R,3S)-1-(Cyclohexylmethyl)-3-cyclopropyl-2,3-dihydroxypropyl]-8-propyl-6-(3-pyridyl)-α-(3-pyridylmethyl)-s-triazolo[3,4-f]-[1,2,4]triazin-3-acetamid,

(S oder R)-N-[(1S,2R,3S)-1-(Cyclohexylmethyl)-3-cyclopropyl-2,3-dihydroxypropyl]-8-isobutyl-6-(3-pyridyl)-α-(3-pyridylmethyl)-s-triazolo[3,4-f]-[1,2,4]triazin-3-acetamid und

(R oder S)-N-[(1S,2R,3S)-1-(Cyclohexylmethyl)-3-cyclopropyl-2,3-dihydroxypropyl]-8-isobutyl-6-(3-pyridyl)-α-(3-pyridylmethyl)-s-triazolo[3,4-f]-[1,2,4]triazin-3-acetamid.

Die Verbindungen der Formel I in Form von optisch reinen Diastereomeren, Diastereomerengemischen, diastereomeren Racematen oder Gemischen von diastereomeren Racematen sowie pharmazeutisch verwendbare Salze davon können hergestellt werden, indem man

a) eine Verbindung der allgemeinen Formel

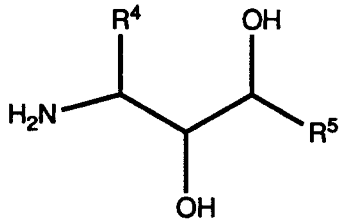

II

worin $R^4$ und $R^5$ die oben angegebene Bedeutung besitzen, mit einer Verbindung der allgemeinen Formel

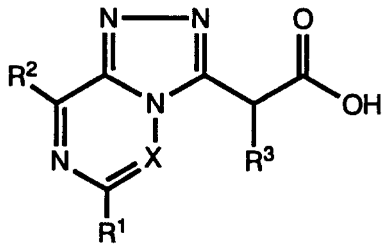

III

worin X, $R^1$, $R^2$ und $R^3$ die oben angegebene Bedeutung besitzen, oder einem aktivierten Derivat davon umsetzt, und

b) erwünschtenfalls eine erhaltene Verbindung der Formel I, worin $R^2$ Alkylthioalkyl bedeutet, zur entsprechenden Verbindung der Formel I, worin $R^2$ Alkylsulfonylalkyl bedeutet, oxidiert, und/oder

c) erwünschtenfalls ein Gemisch diastereomerer Racemate in die diastereomeren Racemate oder optisch reinen Diastereomeren auftrennt, und/oder

d) erwünschtenfalls ein Diastereomerengemisch in die optisch reinen Diastereomeren auftrennt, und/oder

e) erwünschtenfalls eine erhaltene Verbindung in ein pharmazeutisch verwendbares Salz überführt.

Die Acylierung einer Verbindung der Formel II erfolgt nach an sich bekannten Methoden. Geeignete Acylierungsmittel sind insbesondere aktivierte Säurederivate, wie Ester, gemischte Ester, Säurehalogenide und Säureanhydride oder gemischte Säureanhydride. Die Reaktion wird in einem unter den Reaktionsbedingungen inerten organischen Lösungsmittel oder Lösungsmittelgemisch bei einer Temperatur zwischen etwa 0 ° C und der Raumtemperatur durchgeführt. Als Lösungsmittel kommen insbesondere aromatische Kohlenwasserstoffe, wie Benzol, Toluol oder Xylol, chlorierte Kohlenwasserstoffe, wie Methylenchlorid oder Chloroform, Aether, wie Diäthyläther, Tetrahydrofuran oder Dioxan, und dergleichen in Frage. Die Reaktion erfolgt unter in der Peptidchemie üblichen Reäktionsbedingungen. d.h. vorzugsweise in Gegenwart eines Kondensationsmittels wie HBTU (O-Benzotriazolyl-N,N,N',N'-tetramethyluronium-hexafluorophosphat),BOP (Benzotriazol-1-yloxy-tris-(dimethylamino)phosphoniumhexafluorophosphat), BOPC (Bis(2-oxo-3-oxazolidinyl)phosphinsäurechlorid), HOBT (N-Hydroxybenzotriazol), DCC (Dicyclohexylcarbodiimid), EDC (N-Aethyl-N'(3-dimethylaminopropyl)carbodiimid-hydrochlorid), und dergleichen. Die Reaktion wird zweckmässigerweise in einem unter den Reaktionsbedingungen inerten organischen Lösungsmittel oder Lösungsmittelgemisch bei einer Temperatur zwischen etwa 0 ° und 50 ° C, vorzugsweise bei etwa Raumtemperatur, durchgeführt. Als Lösungsmittel kommen insbesondere Dimethylformamid, Methylenchlorid. Acetonitril, Tetrahydrofuran, und dergleichen in Frage.

Die Oxidation einer Verbindung der Formel I, worin $R^2$ Alkylthioalkyl bedeutet, erfolgt ebenfalls nach an sich bekannten Methoden. Die Oxidation kann z.B. durch Umsetzen mit Wasserstoffperoxid, einer organischen Persäure, wie Peressigsäure, Perbenzoesäure, Perphthalsäure und dergleichen, einem Perester, Natriumperjodat, Kaliumperoxomonosulfat in Alkanol/-Wasser und dergleichen, erfolgen. Die Oxidation wird zweckmässigerweise in einem inerten organischen Lösungsmittel, wie einem halogenierten Kohlenwasserstoff, z.B. Chloroform, Methylenchlorid, Dichloräthan und dergleichen, durchgeführt. Wenn Wassestoffperoxid als Oxidationsmittel verwendet wird, kann die Reaktion auch in Essigsäure und dergleichen durchgeführt werden. Der Einfachheit halber wird die Oxidation bei einer Temperatur zwischen etwa 0 ° und 30 ° C, vorzugsweise bei etwa 0 ° C, durchgeführt.

Die Ausgangsstoffe der Formel II sind bekannt oder können in Analogie zur Herstellung der bekannten Verbindungen erhalten werden.

Die Ausgangsstoffe der Formel III, worin X -CH- bedeutet, sind aus EP-A 0.369.743 bekannt oder können in Analogie zur Herstellung der bekannten Verbindungen erhalten werden.

Die übrigen Verbindungen der Formel III sind neu und Gegenstand vorliegender Erfindung. Sie können nach verschiedenen, an sich bekannten, teilweise zum in EP-A 0.369.743 beschriebenen Verfahren analogen Methoden ausgehend von entsprechenden 1,2,4-Triazinonen hergestellt werden. Diese Herstellungsverfahren sowie das Verfahren gemäss EP-A 0.369.743 sind in den nachfolgenden Schemata I und II zusammengefasst. In diesen Schemata bedeutet R Alkyl und besitzen $R^1$, $R^2$ und $R^3$ die oben angegebene Bedeutung. Die 1,2,4-Triazinone der Formel IX in Schema II können in Analogie zur in J. Heterocyclic Chem., 15, 1271 (1978) beschriebenen Herstellung von 1,2,4-Triazinonen erhalten werden.

## Schema I

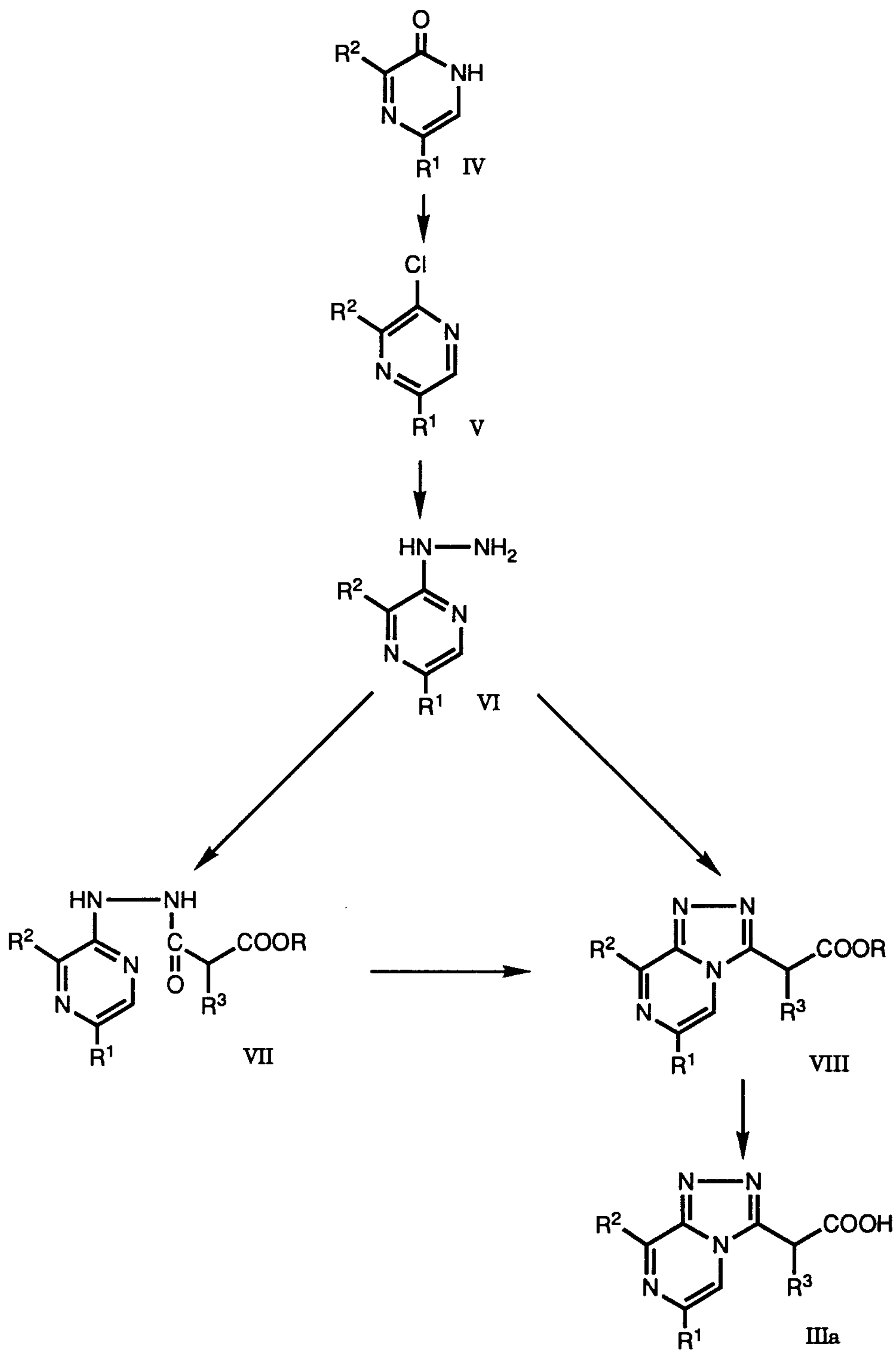

IV

V

VI

VII

VIII

IIIa

## Schema II

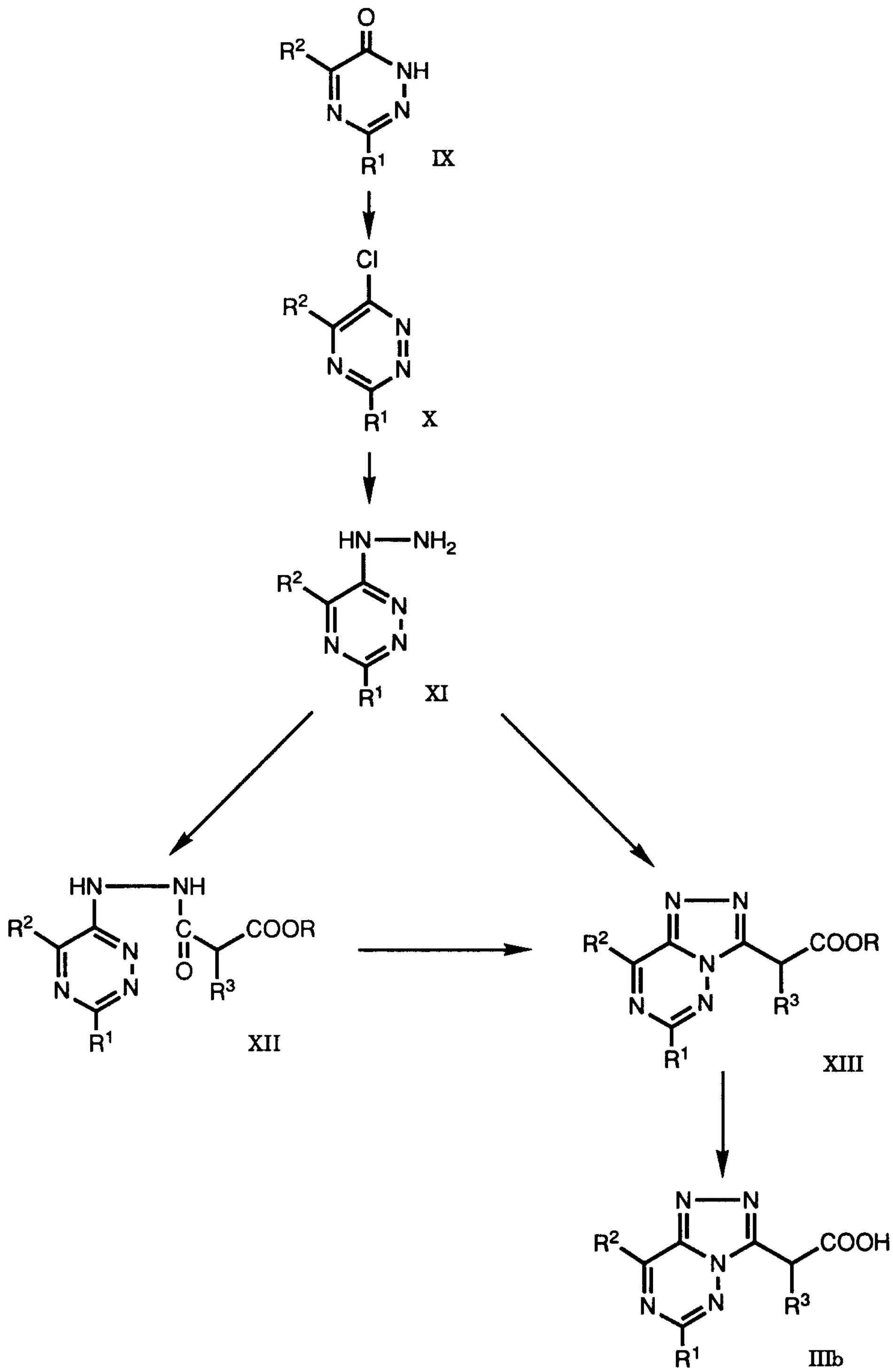

IX

X

XI

XII

XIII

IIIb

Die Verbindungen der Formel I und ihre pharmazeutisch verwendbaren Salze weisen hemmende Wirkung des natürlichen Enzyms Renin auf. Letzteres gelangt aus den Nieren in das Blut und bewirkt dort die Spaltung von Angiotensinogen unter Bildung des Dekapeptids Angiotensin I, das dann in der Lunge, den Nieren und anderen Organen zum Octapeptid Angiotensin II gespalten wird. Angiotensin II erhöht den Blutdruck sowohl direkt durch arterielle Konstriktion, als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II selber oder des daraus als Spaltprodukt gebildeten Heptapeptids Angiotensin III zurückzuführen. Hemmer der enzymatischen Aktivität von Renin bewirken eine Verringerung der Bildung von Angiotensin I und als Folge davon die Bildung einer geringeren Menge von Angiotensin II. Die verminderte Konzentration dieses aktiven Peptid-Hormons ist die unmittelbare Ursache für die blutdrucksenkende Wirkung von Renin-Hemmern.

Die Wirkung von Renin-Hemmern kann experimentell mittels des nachstehend beschriebenen In vitro-Tests gezeigt werden:

In vitro-Test mit reinem Human-Renin

Der Test wird in Eppendorf Röhrchen durchgeführt. Die Inkubationsmischung besteht aus (1) 100 µl Human-Renin in Puffer A (0,1M Natriumphosphatlösung, pH 7.4, enthaltend 0,1% Rinderserumalbumin, 0,1% Natriumazid und 1 mM Aethylendiamintetraessigsäure), genügend für eine Renin-Aktivität von 2-3 ng Angiotensin I/ ml/Std.; (2) 145 µl Puffer A; (3) 30 µl von 10 µM humanem Tetradekapeptid-Reninsubstrat (hTD) in 10 mM Salzsäure; (4) 15 µl Dimethylsulfoxid mit bzw. ohne Hemmer und (5) 10 µl einer 0,03 molaren Lösung von Hydroxychinolinsulfat in Wasser.

Die Proben werden drei Stunden bei 37°C bzw. 4°C in Triplikaten inkubiert. 2 x 100 µl Proben pro Versuchsröhrchen werden dann verwendet, um die Produktion von Angiotensin I via RIA (standard radioimmunoassay; Clinical Assay solid phase kit) zu messen. Kreuzreaktivitäten der verwendeten Antikörper im RIA sind: Angiotensin I 100%; Angiotensin II 0,0013%; hTD (Angiotensin I-Val-Ile-His-Ser-OH) 0,09%. Die Produktion von Angiotensin I wird durch die Differenz zwischen dem Versuch bei 37°C und demjenigen bei 4°C bestimmt.

Folgende Kontrollen werden mitgeführt:

(a) Inkubation von hTD-Proben ohne Renin und ohne Hemmer bei 37°C und 4°C. Die Differenz zwischen diesen beiden Werten ergibt den Grundwert der Angiotensin I-Produktion.

(b) Inkubation von hTD-Proben mit Renin, jedoch ohne Hemmer bei 37°C und 4°C. Die Differenz zwischen diesen Werten ergibt den Maximalwert der Angiotensin I-Produktion.

In jeder Probe wird von der ermittelten Angiotenin I-Produktion der Grundwert der Angiotensin I-Produktion abgezogen. Die Differenz zwischen dem Maximalwert und dem Grundwert ergibt den Wert der maximalen Substrathydrolyse (=100%) durch Renin.

Die Resultate werden als $IC_{50}$-Werte angegeben, welche diejenige Konzentration des Hemmers bezeichnen, bei welcher die enzymatische Aktivität um 50% gehemmt wird. Die $IC_{50}$-Werte werden aus einer linearen Regressionskurve aus einem logit-log plot ermittelt.

Die in diesem Test erhaltenen Resultate sind in der folgenden Tabelle zusammengefasst:

Tabelle

| Verbindung | $IC_{50}$ – Werte in nMol/lt. |
|---|---|
| A | 51 |
| B | 2,0 |
| C | 17 |
| D | 2,3 |
| E | 100 |
| F | 150 |
| G | 16 |

A = (R oder S – N – [(1S,2R,3S) – 1 – (Cyclohexylmethyl) – 3 – cyclopropyl – 2,3 – dihydroxypropyl] – 8 – cyclopropyl – 6 – (3 – pyridyl) – α – (3 – pyridylmethyl – ) – s – triazolo[4,3 – a]pyrazin – 3 – acetamid

B = (R oder S) – N – [(1S,2R,3S) – 1 – (Cyclohexylmethyl) – 3 – cyclopropyl – 2,3 – dihydroxypropyl] – 8 – cyclopropyl – 6 – (3 – pyridyl) – α – (4 – thiazolylmethyl) – s – triazolo[4,3 – a]pyrazin – 3 – acetamid

C = (R oder S) – N – [(1S,2R,3S) – 1 – (Cyclohexylmethyl) – 3 – cyclopropyl – 2,3 – dihydroxypropyl] – 8 – (cyclopropylmethyl) – 6 – (3 – pyridyl) – α – (3 – pyridylmethyl) – s – triazolo[4,3 – a]pyrazin – 3 – acetamid

D = (R oder S) – N – [(1S,2R,3S) – 1 – (Cyclohexylmethyl) – 3 – cyclopropyl – 2,3 – dihydroxypropyl] – 8 – (cyclopropylmethyl) – 6 – (3 – pyridyl) – α – (4 – thiazolylmethyl) – s – triazolo[4,3 – a]pyrazin – 3 – acetamid

E = (R,S) – N – [(1S,2R,3S) – 1 – Benzyl – 3 – cyclopropyl – 2,3 – dihydroxypropyl] – 8 – cyclopropyl – 6 – (3 – pyridyl) – α – (3 – pyridylmethyl) – s – triazolo[4,3 – a]pyrazin – 3 – acetamid

F = (R oder S) – N – [(1S,2R,3S) – 1 – (Cyclohexylmethyl) – 3 – cyclopropyl – 2,3 – dihydroxypropyl] – 8 – isobutyl – 6 – (3 – pyridyl) – α – (3 – pyridylmethyl) – s – triazolo[3,4 – f][1,2,4]triazin – 3 – acetamid

G = (R,S) – α – Allyl – N – [(1S,2R,3S) – 1 – (cyclohexylmethyl)3 – cyclopropyl – – 2,3 – dihydroxypropyl] – 8 – propyl – 6 – (3 – pyridyl) – s – triazolo[4,3 – – a]pyrazin – 3 – acetamid

Die Verbindungen der Formel I sowie deren pharmazeutisch verwendbare Salze können als Heilmittel, z.B. in Form pharmazeutischer Präparate, Verwendung finden. Die pharmazeutischen Präparate können enteral, wie oral, z.B. in Form von Tabletten, Lacktabletten, Dragées, Hart – und Weichgelatinekapseln, Lösungen, Emulsionen oder Suspensionen, nasal, z.B. in Form von Nasensprays, oder rektal, z.B. in Form von Suppositorien, verabreicht werden. Die Verabreichung kann aber auch parenteral, wie intramuskulär oder intravenös, z.B. in Form von Injektionslösungen, erfolgen.

Zur Herstellung von Tabletten, Lacktabletten, Dragées und Hartgelatinekapseln können die Verbindungen der Formel I sowie ihre pharmazeutisch verwendbaren Salze mit pharmazeutisch inerten, anorganischen oder organischen Excipientien verarbeitet werden. Als solche Excipientien kann man z.B. für Tabletten, Dragées und Hartgelatinekapseln, Laktose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose etc.

Für Injektionslösungen eignen sich als Excipientien z.B. Wasser, Alkohole, Polyole, Glycerin, vegetabile Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, viskositätserhöhende Stoffe, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man die Verbindungen der allgemeinen Formel I sowie deren pharmazeutisch verwendbare Salze bei der Bekämpfung bzw. Verhütung von Bluthochdruck und Herzinsuffizienz verwenden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 3 mg bis etwa 3 g, vorzugsweise etwa 10 mg bis etwa 1 g, z.B. ungefähr 300 mg pro Person, verteilt auf vorzugsweise 1 - 3 Einzeldosen, die z.B. gleich gross sein können, angemessen sein, wobei aber die soeben angegebene obere Grenze auch überschritten werden kann, wenn sich dies als angezeigt erweisen sollte. Ueblicherweise erhalten Kinder die halbe Dosis von Erwachsenen.

Die folgenden Beispiele sollen die vorliegende Erfindung erläutern, sie jedoch in keiner Weise einschränken. Alle Temperaturen sind in Celsiusgraden angegeben.

## Beispiel 1

Zu einer Lösung von 0,600 g (1,55 mMol) rac-8-Cyclopropyl-6-(3-pyridyl)-α-(3-pyridylmethyl)-s-triazolo[4,3-a]pyrazin-3-essigsäure und 0,352 g (1,55 mMol) (1S,2R,3S)-3-Amino-4-cyclohexyl-1-cyclopropyl-1,2-butandiol (EP-A 0.332.008) in 30 ml Acetonitril wurden 0,587 g (1,55 mMol) HBTU und 0,215 ml (1,55 mMol) Triäthylamin gegeben, und die erhaltene Suspension 12 Stunden bei Raumtemperatur gerührt. Nach der Zugabe von gesättigter Kochsalzlösung wurde dreimal mit Essigsäureäthylester extrahiert. Die organische Phase wurde mit gesättigter Natriumhydrogencarbonatlösung, Wasser und gesättigter Kochsalzlösung gewaschen, über Magnesiumsulfat getrocknet, filtriert und unter vermindertem Druck eingedampft. Nach Chromatographie an Kieselgel (Methylenchlorid/Methanol, 9:1) erhielt man 0,240 g des reinen, weniger polaren Epimeren (R oder S)-N-[(1S,2R,3S)-1-(Cyclohexylmethyl)-3-cyclopropyl-2,3-dihydroxypropyl]-8-cyclopropyl-6-(3-pyridyl)-α-(3-pyridylmethyl)-s-triazolo[4,3-a]pyrazin-3-acetamid, MS: (FAB) 596 $(M+H)^+$, und 0,160 g des reinen, polareren (S oder R)-N-[(1S,2R,3S)-1-(Cyclohexylmethyl)-3-cyclopropyl-2,3-dihydroxypropyl]-8-cyclopropyl-6-(3-pyridyl)-α-(3-pyridylmethyl)-s-triazolo[4,3-a]pyrazin-3-acetamid, MS: (FAB) 596 $(M+H)^+$, je als farbloser Schaum.

Die als Ausgangsstoff eingesetzte rac-8-Cyclopropyl-6-(3-pyridyl)-α-(3-pyridylmethyl)-s-triazolo[4,3-a]pyrazin-3-essigsäure wurde wie folgt hergestellt:

(a) Zu einer Suspension von 25,18 g (185 mMol) Natriumsalz der 2-Cyclopropyl-2-oxo-essigsäure (Collect. Czech. Chem. Commun. **40**, 1038 (1975)), 38,28 g (185 mMol) 3-(Aminoacetyl)pyridin-dihydrochlorid (J. Chem. Soc. **1938**, 753) und 25,34 g (185 mMol) HOBT in 750 ml Methylenchlorid wurden bei 10° 49g (240 mMol) DCC gegeben und über Nacht bei Raumtemperatur gerührt. Der entstandene Harnstoff wurde abfiltriert, das Filtrat mit gesättigter Natriumhydrogencarbonatlösung und anschliessend mit gesättigter Kochsalzlösung gewaschen, über Magnesiumsulfat getrocknet, filtriert und unter vermindertem Druck eingedampft. Chromatographie an Kieselgel (Methylenchlorid/Methanol, 97:3) lieferte 21 g N-[2-Oxo-2-(3-pyridyl)äthyl]-α-oxocyclopropylacetamid als farblose Kristalle, MS: (EI) 233 $(M+H)^+$.

(b) Eine Lösung von 9,00g (38,76 mMol) N-[2-Oxo-2-(3-pyridyl)äthyl]-α-oxocyclopropylacetamid in 80 ml Äthanol wurde mit 8,90 g (116 mMol) Ammoniumacetat versetzt und 2 Stunden am Rückfluss erhitzt. Man dampfte das Lösungsmittel ein und löste den Rückstand in Methylenchlorid. Die Lösung wurde nacheinander mit Wasser, gesättigter Natriumhydrogencarbonatlösung, Wasser und gesättigter Kochsalzlösung gewaschen, über Magnesiumsulfat getrocknet, filtriert und unter vermindertem Druck eingedampft. Nach Chromatographie an Kieselgel (Methylenchlorid/Methanol, 9:1) und Umkristallisation aus Diäthyläther erhielt man 6,00 g 3-Cyclopropyl-5-(3-pyridyl)-2-(1H)pyrazinon als gelbes Pulver, Smp. 168-170°, MS: (EI) 213 $M^+$.

(c) Eine Lösung von 6,00g (28,13 mMol) 3-Cyclopropyl-5-(3-pyridyl)-2(1H)-pyrazinon in 50 ml Phosphoroxychlorid wurde 2 Stunden zum Rückfluss erhitzt. Die hellbraune Lösung wurde am Rotationsverdampfer unter vermindertem Druck eingedampft, und der Rückstand auf Eiswasser gegossen. Nach Extraktion mit Methylenchlorid, Trocknen über Natriumsulfat, Filtrieren und Eindampfen unter

vermindertem Druck erhielt man 5,20 g 2-Chlor-3-cyclopropyl-5-(3-pyridyl)pyrazin als farbloses Pulver, MS: (EI) 230 $M^+$.

(d) Eine Lösung von 5,20 g (22,4 mMol) 2-Chlor-3-cyclopropyl-5-(3-pyridyl)-pyrazin in 50 ml Äthanol wurde mit 7 mi (140 mMol) Hydrazinhydrat versetzt und 7 Stunden zum Rückfluss erhitzt. Nach dem Abkühlen auf Raumtemperatur nutschte man das ausgefallene Produkt ab und erhielt so 2,70 g 3-Cyclopropyl-2-hydrazino-5-(3-pyridyl)pyrazin als gelb-glänzende Plättchen, Smp. 179-181°, MS: (EI) 227 $M^+$.

(e) Zu einer Lösung von 0,900 g (4 mMol) 3-Cyclopropyl-2-hydrazino-5-(3-pyridyl)pyrazin und 1,5 g (6 mMol) Diäthyl (3-pyridylmethyl)malonat (Arch. Pharm. **308**, 663 (1975)) in 70 ml Xylol gab man 0,760g (4 mMol) p-Toluolsulfonsäure und erhitzte 2 Stunden zum Rückfluss am Wasserabscheider. Nach Abdestillieren des Xylols unter vermindertem Druck löste man den Rückstand in Methylenchlorid. Die organische Phase wurde mit gesättigter Natriumbicarbonatlösung und anschliessend mit gesättigter Kochsalzlösung gewaschen, über Magnesiumsulfat getrocknet, filtriert und unter reduziertem Druck eingedampft. Chromatographie an Kieselgel (Methylenchlorid/Methanol, 13:1) lieferte 0,800 g rac-8-Cyclopropyl-6-(3-pyridyl)-α-(3-pyridylmethyl)-s-triazolo[4,3-a]pyrazin-3-essigsäureäthylester als farbloses Öl (Rf: 0,33, Kieselgel, Methylenchlorid/Methanol, 9:1).

(f) Eine Lösung von 0,300 g (0,724 mMol) rac-8-Cyclopropyl-6-(3-pyridyl)-α-(3-pyridylmethyl)-s-triazolo[4,3-a]pyrazin-3-essigsäureäthylester in 20 ml Äthanol versetzte man mit 10 ml 2N Natronlauge und rührte 6 Stunden bei Raumtemperatur. Danach gab man 10 ml 2N Salzsäure dazu, dampfte ein und verteilte den Rückstand zwischen 10 ml Wasser und 50 ml eines 10:1 Gemisches von Methylenchlorid und Methanol. Die organische Phase wurde über Natriumsulfat getrocknet, filtriert und unter reduziertem Druck eingedampft. Man erhielt so 0,258 g rac-8-Cyclopropyl-6-(3-pyridyl)-α-(3-pyridylmethyl)-s-triazolo[4,3-a]pyrazin-3-essigsäure als farbloses, amorphes Pulver, MS: (EI) 342 $(M-CO_2)^+$.

## Beispiel 2

In analoger Weise wie in Beispiel 1 beschrieben, wurden die folgenden Verbindungen hergestellt:

- Aus rac-8-Cyclopropyl-6-(3-pyridyl)-α-(4-thiazolylmethyl)-s-triazolo[4,3-a]-pyrazin-3-essigsäure und (1S,2R,3S)-3-Amino-4-cyclohexyl-1-cyclopropyl-1,2-butandiol das (R oder S)-N-[(1S,2R,3S)-1-(Cyclohexylmethyl)-3-cyclopropyl-2,3-dihydroxypropyl]-8-cyclopropyl-6-(3-pyridyl)-α-(4-thiazolylmethyl)-s-triazolo[4,3-a]pyrazin-3-acetamid, MS: (FAB) 602 $(M+H)^+$, und das (S oder R)-N-[(1S,2R,3S)-1-(Cyclohexylmethyl)-3-cyclopropyl-2,3-dihydroxypropyl]-8-cyclopropyl-6-(3-pyridyl)-α-(4-thiazolylmethyl)-s-triazolo[4,3-a]pyrazin-3-acetamid, MS: (FAB) 602 $(M+H)^+$, je als farbloser Schaum;
- aus 8-Cyclopropyl-6-(3-pyridyl)-s-triazolo[4,3-a]pyrazin-3-essigsäure und (1S,2R,3S)-3-Amino-4-cyclohexyl-1-cyclopropyl-1,2-butandiol das N-[(1S,2R,3S)-1-(Cyclohexylmethyl)-3-cyclopropyl-2,3-dihydroxypropyl]-8-cyclopropyl-6-(3-pyridyl)-s-triazolo[4,3-a]pyrazin-3-acetamid, MS: (FAB) 505 $(M+H)^+$, als farbloser Schaum;
- aus rac-8-(Cyclopropylmethyl)-6-(3-pyridyl)-α-(3-pyridylmethyl)-s-triazolo-[4,3-a]-pyrazin-3-essigsäure und (1S,2R,3S)-3-Amino-4-cyclohexyl-1-cyclopropyl-1,2-butandiol das (R oder S)-N-[(1S,2R,3S)-1-(Cyclohexylmethyl)-3-cyclopropyl-2,3-dihydroxypropyl]-8-(cyclopropylmethyl)-6-(3-pyridyl)-α-(3-pyridylmethyl)-s-triazolo[4,3-a]pyrazin-3-acetamid, MS: (FAB) 610 $(M+H)^+$, und das (S oder R)-N-[(1S,2R,3S)-1-(Cyclohexylmethyl)-3-cyclopropyl-2,3-dihydroxypropyl]-8-(cyclopropylmethyl)-6-(3-pyridyl)-α-(3-pyridylmethyl)-s-triazolo[4,3-a]pyrazin-3-acetamid, MS: (FAB) 610 $(M+H)^+$, je als farbloser Schaum;
- aus rac-8-(Cyclopropylmethyl)-6-(3-pyridyl)-α-(4-thiazolylmethyl)-s-triazolo[4,3]pyrazin-3-essigsäure und (1S,2R,3S)-3-Amino-4-cyclohexyl-1-cyclopropyl-1,2-butandiol das (R oder S)-N-[(1S,2R,3S)-1-(Cyclohexylmethyl)-3-cyclopropyl-2,3-dihydroxypropyl]-8-(cyclopropylmethyl)-6-(3-pyridyl)-α-(4-thiazolylmethyl)-s-triazolo[4,3-a]pyrazin-3-acetamid, MS: (ISP) 616 $(M+H)^+$, und das (S oder R)-N-[(1S,2R,3S)-1-(Cyclohexylmethyl)-3-cyclopropyl-2,3-dihydroxypropyl]-8-(cyclopropylmethyl)-6-(3-pyridyl)-α-(4-thiazolylmethyl)-s-triazolo[4,3-a]pyrazin-3-acetamid, MS: (ISP) 616 $(M+H)^+$, je als farbloser Schaum;
- aus 8-(Cyclopropylmethyl)-6-(3-pyridyl)-s-triazolo[4,3-a]pyrazin-3-essigsäure und (1S,2R,3S)-3-Amino-4-cyclohexyl-1-cyclopropyl-1,2-butandiol das N-[(1S,2R,3S)-1-(Cyclohexylmethyl)-3-cyclopropyl-2,3-dihydroxypropyl]-8-(cyclopropylmethyl)-6-(3-pyridyl)-s-triazolo[4,3-a]pyrazin-3-acetamid, MS: (ISP) 519 $(M+H)^+$, als farbloser Schaum;

- aus rac-6-(4-Isochinolinyl)-8-propyl-α-(3-pyridylmethyl)-s-triazolo[4,3-a]-pyrazin-3-essigsäure und (1S,2R,3S)-3-Amino-4-cyclohexyl-1-cyclopropyl-1,2-butandiol das (R oder S)-N-[(1S,2R,3S)-1-(Cyclohexylmethyl)-3-cyclopropyl-2,3-dihydroxypropyl]-6-(4-isochinolinyl)-8-propyl-α-(3-pyridylmethyl)-s-triazolo[4,3-a]pyrazin-3-acetamid, MS: (FAB) 648 $(M+H)^+$, und das (S oder R)-N-[(1S,2R,3S)-1-(Cyclohexylmethyl)-3-cyclopropyl-2,3-dihydroxypropyl]-6-(4-isochinolinyl)-8-propyl-α-(3-pyridylmethyl)-s-triazolo[4,3-a]-pyrazin-3-acetamid, MS: (FAB) 648 $(M+H)^+$, je als farbloser Schaum;
- aus 6-(4-Isochinolinyl)-8-propyl-s-triazolo[4,3-a]pyrazin-3-essigsäure und (1S,2R,3S)-3-Amino-4-cyclohexyl-1-cyclopropyl-1,2-butandiol das N-[(1S,2R,3S)-1-(Cyclohexylmethyl)-3-cyclopropyl-2,3-dihydroxypropyl]-6-(4-isochinolinyl)-8-propyl-s-triazolo[4,3-a]pyrazin-3-acetamid, MS: (EI) 556 $M^+$, als farbloser Schaum;
- aus rac-8-Cyclopropyl-6-(3-pyridyl)-α-(3-pyridylmethyl)-s-triazolo[4,3-a]-pyrazin-3-essigsäure und (1S,2R,3S)-3-Amino-1-cyclopropyl-4-phenyl-1,2-butandiol das (R, S)-N-[(1S,2R,3S)-1-Benzyl-3-cyclopropyl-2,3-dihydroxypropyl]-8-cyclopropyl-6-(3-pyridyl)-α-(3-pyridylmethyl)-s-triazolo[4,3-a]pyrazin-3-acetamid, MS: (FAB) 590 $(M+H)^+$ als farbloser Schaum (1:1 Epimerengemisch);
- aus 8-Propyl-6-(3-pyridyl)-s-triazolo[3,4-f][1,2,4]triazin-3-essigsäure und (1S,2R,3S)-3-Amino-4-cyclohexyl-1-cyclopropyl-1,2-butandiol das N-[(1S,2R,3S)-1(Cyclohexylmethyl)-3-cyclopropyl-2,3-dihydroxypropyl]-8-propyl-6-(3-pyridyl)-s-triazolo[3,4-f][1,2,4]triazin-3-acetamid, MS: (FAB) 508 $(M+H)^+$, als farbloser Schaum;
- aus rac-8-Propyl-6-(3pyridyl)-α-(3-pyridylmethyl)-s-triazolo[3,4-f][1,2,4]-triazin-3-essigsäure und (1S,2R,3S)3-Amino-4-cyclohexyl-1-cyclopropyl-1,2-butandiol das (R oder S)-N-[(1S,2R,3S)-1-(Cyclohexylmethyl)-3-cyclopropyl-2,3-dihydroxypropyl]-8-propyl-6-(3-pyridyl)-α-(3-pyridylmethyl)-s-triazolo -[3,4-f][1,2,4]triazin-3-acetamid, MS: (FAB) 599 $(M+H^+)$, und das (S oder R)-N-[(1S,2R,3S)-1-(Cyclohexylmethyl)-3-cyclopropyl-2,3-dihydroxypropyl]-8-propyl-6-(3-pyridyl)-α-(3-pyridylmethyl)-s-triazolo[3,4-f][1,2,4]-triazin-3-acetamid, MS: (FAB) 599 $(M+H)^+$, je als farbloser Schaum;
- aus rac-8-Isobutyl-6-(3-pyridyl)-α-(3-pyridylmethyl)-s-triazolo[3.4-f][1,2,4]-triazin-3-essigsäure und (1S,2R,3S)-3-Amino-4-cyclohexyl-1-cyclopropyl-1,2-butandiol das (R oder S)-N-[(1S,2R,3S)-1-(Cyclohexylmethyl)-3-cyclopropyl-2,3-dihydroxypropyl]-8-isobutyl-6-(3-pyridyl)-α-(3-pyridylmethyl)-s-triazolo-[3,4-f][1,2,4]triazin-3-acetamid, MS: (FAB) 613 $(M+H)^+$, und das (S oder R)-N-[(1S,2R,3S)-1-(Cyclohexylmethyl)-3-cyclopropyl-2,3-dihydroxypropyl]-8-isobuty-6-(3-pyridyl)-α-(3-pyridylmethyl)-s-triazolo[3,4-f][1,2,4]-triazin-3-acetamid, MS: (FAB) 613 $(M+H)^+$, je als farbloser Schaum.

Die als Ausgangsstoffe eingesetzten Säuren wurden wie folgt hergestellt:

rac-8-Cyclopropyl-6-(3-pyridyl)-α-(4-thiazolylmethyl)-s-triazolo[4,3-a]pyrazin-3-essigsäure

In analoger Weise wie in Beispiel 1 (e,f) beschrieben erhielt man durch Kondensation von 3-Cyclopropyl-2-hydrazino-5-(3-pyridyl)pyrazin mit Diäthyl (4-thiazolylmethyl)malonat (EP-A 0.307.837) und anschliessende basische Verseifung die rac-8-Cyclopropyl-6-(3-pyridyl)-α-(4-thiazolylmethyl)-s-triazolo[4,3-a]pyrazin-3-essigsäure als farbloses Pulver (Rf:0,161, Kieselgel, Methylenchlorid/Methanol, 4:1).

8-Cyclopropyl-6-(3-pyridyl)-s-triazolo[4,3-a]pyrazin-3-essigsäure

In analoger Weise wie in Beispiel 1 (e,f) beschrieben, erhielt man durch Kondensation von 3-Cyclopropyl-2-hydrazino-5-(3-pyridyl)pyrazin mit Diäthylmalonat und anschliessende basische Verseifung die 8-Cyclopropyl-6-(3-pyridyl)-s-triazolo[4,3-a]pyrazin-3-essigsäure als farbloses Pulver, MS: (EI) 251 $(M-CO_2)^+$.

rac-8-(Cyclopropylmethyl)-6-(3-pyridyl)-α-(3-pyridylmethyl)-s-triazolo[4,3-a]-pyrazin-3-essigsäure

In analoger Weise wie in Beispiel 1 (a-f) beschrieben erhielt man durch Kopplung von 2-Oxo-3-cyclopropylpropionsäure (J. Med. Chem. **30**, 1074 (1987)) mit 3-(Aminoacetyl)pyridin-dihydrochlorid das N-[2-Oxo-2-(3-pyridyl)-äthyl]-α-oxo-3-cylopropylpropionsäureamid als farbloses Pulver, MS:

(EI) 218 $(M-CO)^+$, welches mit Ammoniumchlorid in Äthanol das 3-(Cyclopropylmethyl)-5-(3-pyridyl)-2(1H)-pyrazinon, MS: (EI) 227 $M^+$, als farbloses Pulver ergab. Chlorierung mit Phosphoroxychlorid lieferte 2-Chlor-3-(cyclopropylmethyl)-5-(3-pyridyl)pyrazin als beiges Pulver, MS: (EI) 245 $M^+$, welches durch Umsetzung mit Hydrazinhydrat das 3-(Cyclopropylmethyl)-2-hydrazino-5-(3-pyridyl)pyrazin als gelbliches Pulver lieferte, Smp. 161-163°, MS: (EI) 241 $M^+$. Kondensation mit Diäthyl (3-pyridylmethyl)malonat und anschliessende basische Verseifung lieferte dann die rac-8-(Cyclopropylmethyl)-6-(3-pyridyl)-α-(3-pyridylmethyl)-s-triazolo[4,3-a]pyrazin-3-essigsäure als farbloses Pulver, MS: (ISN) 400 $(M-H)^-$.

rac-8-(Cyclopropylmethyl)-6-(3-pyridyl)-α-(4-thiazolylmethyl)-s-triazolo-[4,3-a]pyrazin-3-essigsäure

In analoger Weise wie in Beispiel 1 (e,f) beschrieben, erhielt man durch Kondensation von 3-(Cyclopropylmethyl)-2-hydrazino-5-(3-pyridyl)pyrazinmit Diäthyl (4-thiazolylmethyl)malonat und anschliessende basische Verseifung die rac-8-(Cyclopropylmethyl)-6-(3-pyridyl)-α-(4-thiazolylmethyl)-s-triazolo[4,3-a]pyrazin-3-essigsäure als farbloses Pulver, MS: (ISN) 405 $(M-H)^-$.

8-(Cyclopropylmethyl)-6-(3-pyridyl)-s-triazolo[4,3-a]pyrazin-3-essigsäure

In analoger Weise wie in Beispiel 1 (e,f) beschrieben, erhielt man durch Kondensation von 3-(Cyclopropylmethyl)-2-hydrazino-5-(3-pyridyl)pyrazinmit Diäthylmalonat den 8-(Cyclopropylmethyl)-6-(3-pyridyl)-s-triazolo[4,3-a]-pyrazin-3-essigsäureäthylester als farbloses Pulver, Smp. 140-141°, MS: (EI) 337 $M^+$. Anschliessende basische Verseifung ergab die 8-(Cyclopropylmethyl)-6-(3-pyridyl)-s-triazolo[4,3-a]pyrazin-3-essigsäure als farbloses Pulver (Rf:0,12, Methylenchlorid/Methanol, 4:1).

rac-6-(4-Isochinolinyl)-8-propyl-α-(3-pyridylmethyl)-s-triazolo[4,3-a]pyrazin-3-essigsäure

In analoger Weise wie in Beispiel 1 (b-f) beschrieben erhielt man durch Umsetzung von N-[-(Isochinolinylcarbonyl)methyl]-2-oxopentanamid mit Ammoniumchlorid in Äthanol das 5-(4-Isochinolinyl)-3-propyl-2(1H)-pyrazinon als farbloses Pulver, MS: (EI) 265 $M^+$. Chlorierung mit Phosphoroxychlorid lieferte das 4-(5-Chlor-6-propyl-2-pyrazinyl)isochinolin, MS: (EI) 283 $M^+$ als farbloses Pulver, welches durch Umsetzung mit Hydrazinhydrat das 4-(5-Hydrazino-6-propyl-2-pyrazinyl)-isochinolin als gelbliches Pulver lieferte, Smp. 161-163°, MS: (EI) 279 $M^+$. Kondensation mit Diäthyl (3-pyridylmethyl)malonat und anschliessende basische Verseifung lieferte dann die rac-6-(4-Isochinolinyl)-8-propyl-α-(3-pyridylmethyl)-s-triazolo[4,3-a]-pyrazin-3-essigsäure als farbloses Pulver, MS: (EI) 394 $(M-CO_2)^+$.

rac-6-(4-Isochinolinyl)-8-propyl-s-triazolo[4,3-a]pyrazin-3-essigsäure

In analoger Weise wie in Beispiel 1 (e,f) beschrieben erhielt man durch Kondensation von 4-(5-Hydrazino-6-propyl-2-pyrazinyl)isochinolin mit Diäthylmalonat und anschliessende basische Verseifung die rac-6-(4-isochinolinyl)-8-propyl-s-triazolo[4,3-a]pyrazin-3-essigsäure als farbloses Pulver, MS: (EI) 303 $(M-CO_2)^+$.

Das als Ausgangsstoff verwendete N-[(Isochinolinylcarbonyl)methyl]-2-oxopentanamid wurde wie folgt hergestellt:

In analoger Weise wie in Org. Synth. **64,** 19 (1986) für 2,2-Diäthoxy-2-(4-pyridyläthyl)diamin beschrieben, erhielt man, ausgehend von 4-Acetylisochinolin (J. Am. Chem. Soc. **67**, 1268 (1945)), durch Umsetzung mit Hydroxylamin, Herstellung des Tosylates und anschliessende Neber-Umlagerung mit Natriumäthanolat das 2,2-Diäthoxy-2-(4-isochinolinyl)-äthylamin, Smp. 236° (Zersetzung).

Zu einer Suspension von 2,6 g (7,8 mMol) 2,2-Diäthoxy-2-(4-isochinolinyl)äthylamin in 40 ml Methylenchlorid wurden 0,8 ml 2-Oxo-n-valeriansäure, 2,7 mi (15,5 Mol) Triäthylamin und 1,07 g (7,8 mMol) HOBT gegeben, und das Reaktionsgemisch anschliessend mit 1,59 g DCC in 8 ml Methylenchlorid versetzt. Nach 24 Stunden wurde der entstandene Harnstoff abfiltriert, das Filtrat mit gesättigter Natriumhydrogencarbonatlösung und gesättigter Natriumchloridlösung gewaschen, über Magnesiumsulfat getrocknet, filtriert und unter reduziertem Druck eingedampft. Chromatographie an Kieselgel (Methylenchlorid/Äther, 9:1) lieferte 1,5 g eines gelben Öls, welches in 50 ml 2N Salzsäure gelöst und 24 Stunden bei Raumtemperatur gerührt wurde. Das Lösungsmittel wurde unter vermindertem Druck eingedampft, und der so erhaltene

Rückstand mehrmals mit Toluol azeotrop getrocknet. Man erhielt so 1,1 g N-[(4-Isochinolinylcarbonyl)-methyl]2-oxopentanamid-hydrochlorid als hellbraunes Pulver, MS: (EI) 256 $(M-CO)^+$.

rac-8-Propyl-6-(3-pyridyl)-α-(3-pyridylmethyl)-s-triazolo[3,4-f][1,2,4]triazin-3-essigsäure

In analoger Weise wie in Beispiel 1 (c-f) beschrieben, erhielt man aus 5-Propyl-3-(3-pyridyl)-as-triazin-6(1H)-on durch Chlorierung mit Phosphoroxychlorid das 6-Chlor-5-propyl-3-(3-pyridyl)-as-triazin als farbloses Pulver, Smp. 74-76°, MS: (EI) 234 $M^+$, welches durch Umsetzung mit Hydrazin das 6-Hydrazino-5-propyl-3-(3-pyridyl)-as-triazin als gelbes Pulver, MS: (EI) 230 $M^+$, lieferte. Kondensation mit Diäthyl (3-pyridylmethyl)malonat und anschliessende basische Verseifung ergab die rac-8-Propyl-6-(3-pyridyl)-α-(3-pyridylmethyl)-s-triazolo[3,4-f][1,2,4]triazin-3-essigsäure als farbloses Pulver, MS: (FAB) 346 $(M-CO_2+H)^+$.

8-Propyl-6-(3-pyridyl)-s-triazolo[3,4-f][1,2,4]triazin-3-essigsäure

In analoger Weise wie in Beispiel 1 (e,f) beschrieben, erhielt man durch Kondensation von 6-Hydrazino-5-propyl-3-(3-pyridyl)-as-triazin mit Diäthylmalonat und anschliessende basische Verseifung die 8-Propyl-6-(3-pyridyl)-s-triazolo[3,4-f][1,2,4]triazin-3-essigsäure als fabloses Pulver, MS: (FAB) 299 $(M+H)^+$.

Das als Ausgangsstoff verwendete 5-Propyl-3-(3-pyridyl)-as-triazin-6(1H)-on wurde wie folgt hergestellt:

Zu einer Suspension von 6,40 g (36 mMol) Methyl pyridin-3-carboximidat (J. Org. Chem. **26**, 412 (1961)) in 10 ml Äther wurden 6,50 g (36 mMol) D,L-Norvalinäthylester-hydrochlorid in 10 ml Wasser gegeben und 3 Stunden bei Raumtemperatur gerührt. Nach Zugabe von 50 ml Methylenchlorid wurde die organische Phase abgetrennt, über Natriumsulfat getrocknet, filtriert und unter vermindertem Druck eingedampft. Das so erhaltene hellgelbe Öl (6,05 g) wurde mit 1,5 ml (30 mMol) Hydrazinhydrat in 20 ml Isopropanol 2 Stunden zum Rückfluss erhitzt. Nach Abkühlen auf Raumtemperatur versetzte man mit 10 ml Wasser und extrahierte mit Methylenchlorid. Die organischen Phasen wurden über Magnesiumsulfat getrocknet, filtriert und unter vermindertem Druck eingedampft. Nach Chromatographie an Kieselgel (Methylenchlorid/Methanol, 9:1) erhielt man 2,50 g rac2,5-Dihydro-5-propyl-3-(3-pyridyl)-as-triazin-6(1H)-on als farbloses Pulver, Smp. 144-146°, MS: (EI) 218 $M^+$.

Zu einer Lösung von 1,09 g (5 mMol) rac-2,5-Dihydro-5-propyl-3-(3-pyridyl)-as-triazin-6-(1H)-on in 10 ml 1N Natronlauge wurden 6,4 ml (10,5 mMol) 10 %ige Javellauge unter Eiskühlung gegeben und anschliessend 12 Stunden bei Raumtemperatur gerührt. Extraktion mit Methylenchlorid, Trocknen über Natriumsulfat, Filtrieren und Eindampfen unter vermindertem Druck lieferte 0,58 g 5-Propyl-3-(3-pyridyl)-as-triazin-6(1H)-on als farbloses Pulver, Smp. 160-162°, MS: (EI) 216 $M^+$.

rac-8-Isobutyl-6-(3-pyridyl)-α-(3-pyridylmethyl)-s-triazolo[3,4-f][1,2,4]triazin-3-ssigsäure

In analoger Weise wie in Beispiel 1 (c-f) beschrieben erhielt man aus 5-Isobutyl-3-(3-pyridyl)-as-triazin-6(1H)-on durch Chlorierung mit Phosphoroxychlorid das 6-Chlor-5-isobutyl-3-(3-pyridyl)-as-triazin als farbloses Pulver, Smp. 63-65°, MS: (EI) 248 $M^+$, welches durch Umsetzung mit Hydrazin das 6-Hydrazino-5-isobutyl-3-(3-pyridyl)-as-triazin als gelbes Pulver lieferte, Smp. 126-129°, MS: (EI) 244 $M^+$. Kondensation mit Diäthyl (3-pyridylmethyl)-malonat und anschliessende basische Verseifung ergab die rac-8-Isobutyl-6-(3-pyridyl)-α-(3-pyridylmethyl)-s-triazolo[3,4-f][1,2,4]triazin-3-essigsäure als farbloses Pulver, MS:(FAB) 359 $(M-CO_2+H)^+$.

Das als Ausgangsstoff verwendete 5-Isobutyl-3-(3-pyridyl)-as-triazin-6(1H)-on wurde wie folgt hergestellt:

In analoger Weise wie für das 5-Propyl-3-(3-pyridyl)-as-triazin-6(1H)-on beschrieben erhielt man bei Verwendung von D,L-Leucinäthylester-hydrochlorid anstelle des D,L-Norvalinäthylester-hydrochlorids das 5-Isobutyl-3-(3-pyridyl)-as-triazin-6(1H)-on als farbloses Pulver, Smp. 141-144°, MS: (EI) 230 $M^+$.

Das als Ausgangsstoff eingesetzte (1S,2R,3S)-3-Amino-1-cyclopropyl-4-phenyl-1,2-butandiol wurde wie folgt hergestellt:

(1S,2R,3S)-3-Amino-1-cyclopropyl-4-phenyl-1,2-butandiol

Zu 3,03 g (124,5 mMol) Magnesium in 10 ml abs. Äther tropfte man innert 30 Minuten unter leichtem Rückfluss 9,96 ml (124,5 mMol) Cyclopropylbromid in 100 ml Äther und erhitzte anschliessend 2 1/2 Stunden zum Rückfluss. Anschliessend fügte man innerhalb von 45 Minuten 6,25 g (22,63 mMol) tert.-Butyl [1S,2R)-1-benzyl-2-cyano-2-hydroxyäthyl]carbamat (EP-A 0.266.950) unter Rückfluss tropfenweise zu und erhitzte noch weitere 2 1/2 Stunden zum Rückfluss. Schliesslich liess man abkühlen (10°), tropfte 10%ige Zitronensäure zu und extrahierte zweimal mit Äther. Die organische Phase wurde mit Wasser und gesättigter Kochsalzlösung gewaschen, über Magnesiumsulfat getrocknet, filtriert und unter vermindertem Druck eingedampft. Chromatographie an Kieselgel (Toluol/Essigester, 9:1) lieferte das tert-Butyl [(1S,2R)-1-benzyl-2-(cyclopropylcarbonyl)-2-hydroxyäthyl]-carbamat als hellgelben Feststoff, MS: (EI) 246 $(M-C_3H_9O)^+$.

Eine Lösung von 2,83 g (8,77 mMol) tert-Butyl [(1S,2R)-1-benzyl-2-(cyclopropylcarbonyl)-2-hydroxyäthyl]carbamat löste man in 130 ml Methylenchlorid, fügte 3 ml Essigsäure hinzu und versetzte schliesslich portionsweise bei 0 - 10° mit 0,332 mg (8,78 mMol) Natriumborhydrid. Man rührte weitere 2 Stunden bei 5° und verteilte die Reaktionslösung zwischen 2N Natriumhydrogencarbonatlösung und Methylenchlorid. Die organische Phase wurde abgetrennt, mit Wasser und mit gesättigter Kochsalzlösung gewaschen, über Magnesiumsulfat getrocknet, filtiert und unter vermindertem Druck eingedampft. Kristallisation des Rückstandes aus Äther/Hexan lieferte 2,1g tert-Butyl [(1S,2R,3S)-1-benzyl-3-cyclopropyl-2,3-dihydroxy-propyl]carbamat als farblose Nadeln, Smp. 83-85°.

Eine Lösung von 2,0 g (6,23 mMol) tert-Butyl [(1S,2R,3S)-1-benzyl-3-cyclopropyl-2,3-dihydroxypropyl]carbamat in 20 ml Methanol versetzte man mit 20 ml 2N Salzsäure und erhitzte die Lösung 90 Minuten auf 50°. Nach dem Abkühlen neutralisierte man durch Zugabe von 40 ml 1N Natronlauge und engte am Rotationsverdampfer unter vermindertem Druck zur Trockene ein. Den Rückstand verteilte man zwischen Wasser und Methylenchlorid. Die organische Phase wurde mit Wasser und gesättigter Kochsalzlösung gewaschen, über Magnesiumsulfat getrocknet, filtriert und unter vermindertem Druck eingedampft. Man erhielt so das (1S,2R,3S)-3-Amino-1-cyclopropyl-4-phenyl-1,2-butandiol als weissen, kristallinen Feststoff, MS: (EI) 150 $(M-C_4H_8O)+$.

Beispiel 3

In analoger Weise wie in Beispiel 1 beschrieben, wurden die folgenden Verbindungen hergestellt:

- Aus rac-8-(3-Methylsulfanylpropyl)-6-(3-pyridyl)-α-(3-pyridylmethyl)-s-triazolo[4,3-a]pyrazin-3-essigsäure und (1S,2R,3S)-3-Amino-4-cyclohexyl-1-cyclopropyl-1,2-butandiol das (R oder S)-N-[(1S,2R,3S)-1-(Cyclohexylmethyl)-3-cyclopropyl-2,3-dihydroxypropyl]-8-(3-methylsulfanylpropyl)-6-(3-pyridyl)-α-(3-pyridylmethyl)-s-triazolo[4,3-a]pyrazin-3-acetamid, MS: (FAB) 644 $(M+H)^+$, und das (S oder R)-N-[(1S,2R,3S)-1-(Cyclohexylmethyl)-3-cyclopropyl-2,3-dihydroxypropyl]-8-(3-methylsulfanylpropyl)-6-(3-pyridyl)-α-(3-pyridylmethyl)-s-triazolo[4,3-a]pyrazin-3-acetamid, MS: (FAB) 644 $(M+H)^+$, je als farbloser Schaum;
- aus rac-α-Allyl-8-propyl-6-(3-pyridyl)-s-triazolo[4,3-a]pyrazin-3-essigsäure und (1S,2R,3S)-3-Amino-4-cyclohexyl-1-cyclopropyl-1,2-butandiol das (R,S)-α-Allyl-N-[-(1S,2R,3S)-1-(cyclohexylmethyl)-3-cyclopropyl-2,3-dihydroxypropyl]-8-propyl-6-(3-pyridyl)-s-triazolo[4,3-a]pyrazin-3-acetamid, MS: (FAB) 547, $(M+H)^+$, als farbloser Schaum.

Die als Ausgangsstoffe eingesetzten Säuren wurden wie folgt hergestellt:

rac-8-(3-Methylsulfanylpropyl)-6-(3-pyridyl)-α-(3-pyridylmethyl)-s-triazolo[4,3-a]pyrazin-3-essigsäure

In analoger Weise wie in Beispiel 1 (a-f) beschrieben erhielt man durch Kopplung von 5-(Methylsulfanyl)-2-oxopentansäure (J. Med. Chem. **30**, 1074 (1987)) mit 3-(Aminoacetyl)pyridin-dihydrochlorid das N-[2-Oxo-2-(3-pyridyl)-äthyl]-α-oxo-5-methansulfanylpentansäureamid als farbloses Pulver, MS: (EI) 280 $M^+$, welches mit Ammoniumchlorid in Äthanol das 3-(3-Methansulfanylpropyl)-5-(3-pyridyl)-2(1H)-pyrazinon, MS: (EI) 261 $M^+$, als farbloses Pulver ergab. Chlorierung mit Phosphoroxychlorid lieferte 2-Chlor-3-(3-methylsulfanylpropyl)-5-(3-pyridyl)pyrazin als beiges Pulver, MS: (EI) 244 $(M-Cl)^+$, welches durch Umsetzung mit Hydrazinhydrat das 2-Hydrazino-3-(3-methylsulfanylpropyl)-5-(3-pyridyl)pyrazin als gelbliches Pulver, MS: (EI) 275 $M^+$, lieferte. Kondensation mit Diäthyl (3-pyridylmethyl)malonat und anschliessende basische Verseifung lieferte dann die rac-8-(3−Methylsulfanylpropyl)−6−(3−pyridyl)−α−(3−pyridylmethyl)−s−triazolo[4,3−a]pyrazin−3−essigsäure als farblosen Schaum, MS: (EI) 390 $(M-CO_2)^+$.

rac−α−Allyl−8−propyl−6−(3−pyridyl)−s−triazolo[4,3−a]pyrazin−3−essigsäure.

Zu einer Lösung von 975 mg (3 mMol) 8−Propyl−6−(3−pyridyl)−s−triazolo−[4,3−a]pyrazin−3−essigsäureäthylester (EP−A 0.369.743) in 25 ml Dimethylformamid wurden bei 0° auf einmal 135 mg (3.1 mMol) Natriumhydrid gegeben. Nach der Beendigung der Wasserstoffentwicklung wurden 0.25 ml (3 mMol) Allylbromid in 5 ml Dimethylformamid langsam zugetropft, und die entstandene Lösung 2 Stunden nachgerührt. Die Reaktionslösung wurde auf Eiswasser gegossen und dreimal mit Methylenchlorid extrahiert. Die organische Phase wurde über Magnesiumsulfat getrocknet, filtriert und unter vermindertem Druck eingedampft. Chromatographie an Kieselgel (Methylenchlorid/Methanol 25:1 ) lieferte 800 mg rac−α−Allyl−8−propyl−6−(3−pyridyl)−s−triazolo[4,3−a]pyrazin−3−essigsäureäthylester als farbloses Öl, MS: (EI) 365 $M^+$, welcher in analoger Weise wie in Beispiel 1 (f) beschrieben verseift wurde und so die rac−α−Allyl−8−propyl−6−(3−pyridyl)−s−triazolo[4,3−a]pyrazin−3−essigsäure, MS: (EI) 293 $(M-CO_2)^+$, als farbloses Pulver lieferte.

Beispiel 4

Zu einer Lösung von 100 mg (0.155 mMol) (R oder S)−N−[(1S,2R,3S)−1−(Cyclohexylmethyl)−3−cyclopropyl−2,3−dihydroxypropyl]−8−(3−methylsulfanylpropyl)−6−(3−pyridyl)−α−(3−pyridylmethyl)−s−triazolo[4,3−a]pyrazin−3−acetamid in 2 ml Methylenchlorid wurden bei 0° 62,9 mg (0.311 mMol) m−Chlorperbenzoesäure (85%) gegeben und 12 Stunden bei Raumtemperatur nachgerührt. Die Reaktionslösung wurde mit 10 ml Methylenchlorid verdünnt, anschliessend mit 10% Natriumsulfitlösung, gesättigter Natriumbicarbonatlösung und Wasser gewaschen. Die organische Phase wurde über Magnesiumsulfat getrocknet, filtriert und unter vermindertem Druck eingedampft. Chromatographie an Kieselgel (Methylenchlorid/Methanol/−Ammoniak 110:10:1) lieferte 50 mg (R oder S)−N−[(1S,2R,3S)−1−(Cyclohexylmethyl)−3−cyclopropyl−2,3−dihydroxypropyl]−8−(3−methylsulfonylpropyl)−6−(3−pyridyl)−α−(3−pyridylmethyl)−s−triazolo[4,3−a]pyrazin−3−acetamid als farblose Kristalle, MS: (ISP) 676 $(M+H)^+$.

In analoger Weise wie oben beschrieben erhielt man aus (S oder R)−N−[(1S,2R,3S)−1−(Cyclohexylmethyl)−3−cyclopropyl−2,3−dihydroxypropyl]−8−(3−methylsulfanylpropyl)−6−(3−pyridyl)−α−(3−pyridylmethyl)−s−triazolo[4,3−a]−pyrazin−3−acetamid das (S oder R)−N−[−(1S,2R,3S)−1−(Cyclohexylmethyl)−3−cyclopropyl−2,3−dihydroxypropyl]−8−(3−methylsulfonylpropyl)−6−(3−pyridyl)−α−(3−pyridylmethyl)−s−triazolo[4,3−a]pyrazin−3−acetamid als farblose Kristalle, MS: (ISP) 676 $(M+H)^+$.

Beispiel A

Orale, wässrige Suspension

**Zusammensetzung:**

| | |
|---|---|
| (R oder S)-N-[(1S,2R,3S)-1-(Cyclohexylmethyl)-3-cyclopropyl-2,3-dihydroxypropyl]-8-cyclopropyl-6-(3-pyridyl)-α-(4-thiazolylmethyl)-s-triazolo[4,3-a]pyrazin-3-acetamid mikronisiert | 5.0 g |
| Polysorbat 80 | 0.3 g |
| Hydroxypropylmethylcellulose | 1.0 g |
| Geschmackstoff | q.s. |
| Methylparaben | 0.2 g |
| Propylparaben | 0.04 g |
| Wasser | ad 100.0 ml |

Beispiel B

**Zusammensetzung:**

| | |
|---|---|
| (R oder S)-N-[(1S,2R,3S)-1-(Cyclohexylmethyl)-3-cyclopropyl-2,3-dihydroxypropyl]-8-cyclopropyl-6-(3-pyridyl)-α-(4-thiazolylmethyl)-s-triazolo[4,3-a]pyrazin-3-acetamid-methansulfonat | 1.0 g |
| Methylparaben | 0.2 g |
| Propylparaben | 0.04 g |
| Geschmackstoff | q.s. |
| Wasser | ad 100.0 ml |

Beispiel C

Tabletten

**Zusammensetzung:**

| | | |
|---|---|---|
| 1) | (R oder S)-N-[(1S,2R,3S)-1-(Cyclohexylmethyl)-3-cyclopropyl-2,3-dihydroxypropyl]-8-cyclopropyl-6-(3-pyridyl)-α-(4-thiazolylmethyl)-s-triazolo[4,3-a]pyrazin-3-acetamid-methan-sulfonat | 200 mg |
| 2) | Milchzucker wasserfrei | 160 mg |
| 3) | Hydroxypropylmethylcellulose | 18 mg |
| 4) | Natrium-carboxymethylcellulose | 20 mg |
| 5) | Magnesiumstearat | 2 mg |
| | Tablettengewicht | 400 mg |

Herstellungsverfahren:

1) und 2) werden intensiv gemischt. Die Mischung wird danach mit einer wäßrigen Lösung von 3) befeuchtet und geknetet, und die resultierende Masse granuliert, getrocknet und gesiebt. Das Granulat wird mit 4) und 5) gemischt und zu Tabletten geeigneter Größe verpreßt.

Beispiel D

Kapseln

**Zusammensetzung:**

| | | |
|---|---|---|
| 1) | (R oder S)-N-[(1S,2R,3S)-1-(Cyclohexylmethyl)-3-cyclopropyl-2,3-dihydroxypropyl]-8-cyclopropyl-6-(3-pyridyl)-α-(4-thiazolylmethyl)-s-triazolo[4,3-a]pyrazin-3-acetamid-methan-sulfonat | 200 mg |
| 2) | Milchzucker wasserfrei | 160 mg |
| 3) | Hydroxypropylmethylcellulose | 18 mg |
| 4) | Natrium-carboxymethylcellulose | 20 mg |
| 5) | Magnesiumstearat | 2 mg |
| | Kapselfüllgewicht | 400 mg |

Herstellungsverfahren:

1) und 2) werden intensiv gemischt. Die Mischung wird danach mit einer wäßrigen Lösung von 3) befeuchtet und geknetet, und die resultierende Masse granuliert, getrocknet und gesiebt. Das Granulat wird mit 4) und 5) gemischt, das Gemisch in Kapseln geeigneter Größe abgefüllt.

Beispiel E

Injektionslösung

| Zusammensetzung: | 1 ml |
|---|---|
| (R oder S) – N – [(1S,2R,3S) – 1 – (Cyclohexylmethyl) – 3 – cyclopropyl – 2,3 – dihydroxypropyl] – 8 – cyclopropyl – 6 – (3 – pyridyl) – α – (4 – thiazolylmethyl) – s – triazolo[4,3 – a]pyrazin – 3 – acetamid – – methansulfonat | 20 mg |
| D – Mannit pyrogenfrei | 10 mg |
| Wasser zu Injektionszwecken | ad 1.0ml |

Herstellungsverfahren:

Der Wirkstoff und das Mannit werden in Stickstoff – begastem Wasser gelöst und anschließend nach einem üblichen Verfahren lyophilisiert.

Beispiel F

Wenn man nach den in den Beispielen A – E beschriebenen Verfahren arbeitet, können aus den folgenden, ebenfalls bevorzugten Verbindungen und ihren pharmazeutisch verwendbaren Salzen entsprechende galenische Präparate hergestellt werden:

- (R oder S) – N – [(1S,2R,3S) – 1 – (Cyclohexylmethyl) – 3 – cyclopropyl – 2,3 – dihydroxypropyl] – 8 – cyclopropyl – 6 – (3 – pyridyl) – α – (3 – pyridylmethyl) – s – triazolo[4,3 – a]pyrazin – 3 – acetamid,
- (R oder S) – N – [(1S,2R,3S) – 1 – (Cyclohexylmethyl) – 3 – cyclopropyl – 2,3 – dihydroxypropyl] – 8 – (cyclopropylmethyl) – 6 – (3 – pyridyl) – α – (3 – pyridylmethyl) – s – triazolo[4,3 – a]pyrazin – 3 – acetamid,

  (R oder S) – N – [(1S,2R,3S) – 1 – (Cyclohexylmethyl) – 3 – cyclopropyl – 2,3 – dihydroxypropyl] – 8 – (cyclopropylmethyl) – 6 – (3 – pyridyl) – α – (4 – thiazolylmethyl) – s – triazolo[4,3 – a]pyrazin – 3 – acetamid.

## Patentansprüche

**1.** Aminosäurederivate der allgemeinen Formel

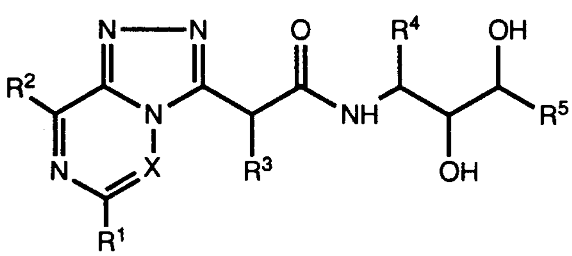

I

worin X ein Stickstoffatom oder – CH – , $R^1$ Phenyl, Pyridyl oder Isochinolinyl, $R^2$ Cycloalkyl, Cycloalkylalkyl, Alkylthioalkyl, Alkylsulfonylalkyl, Alkenyl oder, falls X ein Stickstoffatom und/oder $R^1$ Isochinolinyl und/oder $R^3$ Alkenyl bedeuten, zusätzlich Alkyl, $R^3$ Wasserstoff, Alkyl, Alkenyl, Imidazolylmethyl, Pyridylmethyl, Thiazolylmethyl oder Benzyl, $R^4$ Cyclohexylmethyl oder Benzyl und $R^5$ Cycloalkyl, Alkyl oder Heterocyclylalkyl bedeuten,
in Form von optisch reinen Diastereomeren, Diastereomerengemischen, diastereomeren Racematen oder Gemischen von diastereomeren Racematen sowie pharmazeutisch verwendbare Salze dieser Verbindungen.

2. Verbindungen gemäss Anspruch 1, worin $R^2$ Cycloalkyl, Cycloalkylalkyl, Alkylthioalkyl, Alkylsulfonylalkyl, Alkenyl oder, falls X ein Stickstoffatom und/oder $R^1$ Isochinolinyl bedeuten, zusätzlich Alkyl bedeutet.

3. Verbindungen gemäss Anspruch 1 oder 2, worin X –CH– bedeutet.

4. Verbindungen gemäss einem der Ansprüche 1–3, worin $R^1$ Pyridyl, vorzugsweise 3-Pyridyl, bedeutet.

5. Verbindungen gemäss einem der Ansprüche 1–4, worin $R^2$ Cycloalkyl oder Cycloalkylalkyl, vorzugsweise Cycloalkyl, insbesondere Cyclopropyl, bedeutet.

6. Verbindungen gemäss einem der Ansprüche 1–5, worin $R^3$ Imidazolylmethyl, Pyridylmethyl oder Thiazolylmethyl, vorzugsweise Pyridylmethyl oder Thiazolylmethyl, insbesondere 4-Thiazolylmethyl, bedeutet.

7. Verbindungen gemäss einem der Ansprüche 1–6, worin $R^4$ Cyclohexylmethyl bedeutet.

8. Verbindungen gemäss einem der Ansprüche 1–7, worin $R^5$ Cycloalkyl oder Alkyl, vorzugsweise Cycloalkyl, insbesondere Cyclopropyl, bedeutet.

9. Verbindungen gemäss einem der Ansprüche 1–8, worin X –CH–, $R^1$ 3-Pyridyl, $R^2$ Cyclopropyl, $R^3$ 4-Thiazolylmethyl, $R^4$ Cyclohexylmethyl und $R^5$ Cyclopropyl bedeuten.

10. (R oder S)-N-[(1S,2R,3S)-1-(Cyclohexylmethyl)-3-cyclopropyl-2,3-dihydroxypropyl]-8-cyclopropyl-6-(3-pyridyl)-α-(3-pyridylmethyl)-s-triazolo-[4,3-a]pyrazin-3-acetamid.

11. N-[(1S,2R,3S)-1-(Cyclohexylmethyl)-3-cyclopropyl-2,3-dihydroxypropyl]-8-cyclopropyl-6-(3-pyridyl)-s-triazolo[4,3-a]pyrazin-3-acetamid.

12. (R oder S)-N-[(1S,2R,3S)-1-(Cyclohexylmethyl)-3-cyclopropyl-2,3-dihydroxypropyl]-8-cyclopropyl-6-(3-pyridyl)-α-(4-thiazolylmethyl)-s-triazolo[4,3-a]pyrazin-3-acetamid.

13. (R,S)-N-[(1S,2R,3S)-1-Benzyl-3-cyclopropyl-2,3-dihydroxypropyl]-8-cyclopropyl-6-(3-pyridyl)-α-(3-pyridylmethyl)-s-triazolo[4,3-a]pyrazin-3-acetamid.

14. (R oder S)-N-[(1S,2R,3S)-1-(Cyclohexylmethyl)-3-cyclopropyl-2,3-dihydroxypropyl]-8-(cyclopropylmethyl)-6-(3-pyridyl)-α-(3-pyridylmethyl)-s-triazolo[4,3-a]pyrazin-3-acetamid.

15. (R oder S)-N-[(1S,2R,3S)-1-(Cyclohexylmethyl)-3-cyclopropyl-2,3-dihydroxypropyl]-8-(cyclopropylmethyl)-6-(3-pyridyl)-α-(4-thiazolylmethyl)-s-triazolo[4,3-a]pyrazin-3-acetamid.

16. (R,S)-α-Allyl-N-[(1S,2R,3S)-1-(cyclohexylmethyl)-3-cyclopropyl-2,3-dihydroxypropyl]-8-propyl-6-(3-pyridyl)-s-triazolo[4,3-a]pyrazin-3-acetamid.

17. N-[(1S,2R,3S)-1-(Cyclohexylmethyl)-3-cyclopropyl-2,3-dihydroxypropyl]-6-(4-isochinolinyl)-8-propyl-s-triazolo[4,3-a]pyrazin-3-acetamid, (S oder R)-N-[(1S,2R,3S)-1-(Cyclohexylmethyl)-3-cyclopropyl-2,3-dihydroxypropyl]-6-(4-isochinolinyl)-8-propyl-α-(3-pyridylmethyl)-s-triazolo[4,3-a]-pyrazin-3-acetamid oder (R oder S)-N-[(1S,2R,3S)-1-(Cyclohexylmethyl)-3-cyclopropyl-2,3-dihydroxypropyl]-6-(4-isochinolinyl)-8-propyl-α-(3-pyridylmethyl)-s-triazolo[4,3-a]pyrazin-3-acetamid.

18. N-[(1S,2R,3S)-1-(Cyclohexylmethyl)-3-cyclopropyl-2,3-dihydroxypropyl]-8-propyl-6-(3-pyridyl)-s-triazolo[3,4-f][1,2,4]triazin-3-acetamid, (R oder S)-N-[(1S,2R,3S)-1-(Cyclohexylmethyl)-3-cyclopropyl-2,3-dihydroxypropyl]-8-propyl-6-(3-pyridyl)-α-(3-pyridylmethyl)-s-triazolo[3,4-f][1,2,4]-triazin-3-acetamid, (s oder R)-N-[(1S,2R,3S)-1-(Cyclohexylmethyl)-3-cyclopropyl-2,3-dihydroxypropyl]-8-propyl-6-(3-pyridyl)-α-(3-pyridylmethyl)-s-triazolo[3,4-f][1,2,4]triazin-3-acetamid, (S oder R)-N-[(1S,2R,3S)-1-(Cyclohexylmethyl)-3-cyclopropyl-2,3-dihydroxypropyl]-8-isobutyl-6-(3-pyridyl)-α-(3-pyridylmethyl)-s-triazolol[3,4f][1,2,4]triazin-3-acetamid oder (R oder S)-N-[(1S,2R,3S)-1-(Cyclohexylmethyl)-3-cyclopropyl-2,3-dihydroxypropyl]-8-isobutyl-6-(3-pyridyl)-α-(3-pyridylmethyl)-s-triazolo[3,4-f][1,2,4]triazin-3-acetamid.

**19.** Verbindungen der allgemeinen Formel

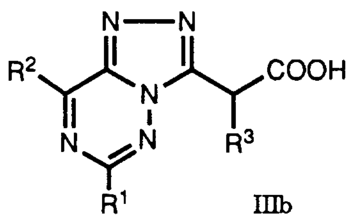

IIIb

worin $R^1$ Phenyl, Pyridyl oder Isochinolinyl, $R^2$ Cycloalkyl, Cycloalkylalkyl, Alkylthioalkyl, Alkylsulfonylalkyl, Alkenyl oder Alkyl und $R^3$ Wasserstoff, Alkyl, Alkenyl, Imidazolylmethyl, Pyridylmethyl, Thiazolylmethyl oder Benzyl bedeuten.

**20.** Aminosäurederivate gemäss einem der Ansprüche 1-18 zur Anwendung als therapeutische Wirkstoffe.

**21.** Aminosäurederivate gemäss einem der Ansprüchr 1-18 zur Anwendung bei der Bekämpfung bzw. Verhütung von Bluthochdruck und Herzinsuffizienz.

**22.** Verfahren zur Herstellung einer Verbindung gemäss einem der Ansprüche 1-18 dadurch gekennzeichnet, dass man

a) eine Verbindung der allgemeinen Formel

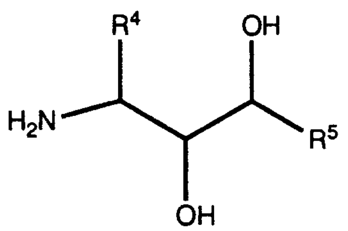

II

worin $R^4$ und $R^5$ die in Anspruch 1 angegebene Bedeutung besitzen,
mit einer Verbindung der allgemeinen Formel

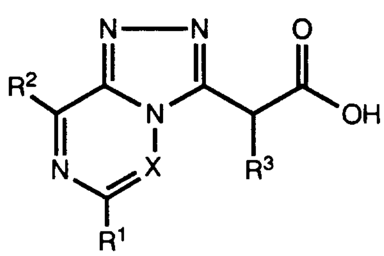

III

worin X, $R^1$, $R^2$ und $R^3$ die in Anspruch 1 angegebene Bedeutung besitzen,
oder einem aktivierten Derivat davon umsetzt, und

b) erwünschtenfalls eine erhaltene Verbindung der Formel I, worin $R^2$ Alkylthioalkyl bedeutet, zur entsprechenden Verbindung der Formel I, worin $R^2$ Alkylsulfonylalkyl bedeutet, oxidiert, und/oder

c) erwünschtenfalls ein Gemisch diastereomerer Racemate in die diastereomeren Racemate oder optisch reinen Diastereomeren auftrennt, und/oder

d) erwünschtenfalls ein Diastereomerengemisch in die optisch reinen Diastereomeren auftrennt, und/oder

e) erwünschtenfalls eine erhaltene Verbindung in ein pharmazeutisch verwendbares Salz überführt.

**23.** Arzneimittel, enthaltend ein Aminosäurederivat gemäss einem der Ansprüche 1 – 18 und ein therapeu – tisch inertes Excipiens.

**24.** Mittel zur Bekämpfung bzw. Verhütung von Bluthochdruck und Herzinsuffizienz, enthaltend ein Amino – säurederivat gemäss einem der Ansprüche 1 – 18 und ein therapeutisch inertes Excipiens.

**25.** Verwendung eines Aminosäurederivates gemäss einem der Ansprüche 1 – 18 bei der Bekämpfung bzw. Verhütung von Krankheiten.

**26.** Verwendung eines Aminosäurederivates gemäss einem der Ansprüche 1 – 18 bei der Bekämpfung bzw. Verhütung von Bluthochdruck und Herzinsuffizienz.

**27.** Verwendung eines Aminosäurederivates gemäss einem der Ansprüche 1 – 18 zur Herstellung von Mitteln gegen Bluthochdruck und/oder Herzinsuffizienz.

Europäisches Patentamt

# EUROPÄISCHER RECHERCHENBERICHT

Nummer der Anmeldung

EP 92 11 9128

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | EP-A-0 332 008 (F. HOFFMANN-LA ROCHE)<br>* Ansprüche 1,23,25 * | 1,24,26 | C07D487/04<br>A61K31/495<br>A61K31/53<br>//(C07D487/04, 249:00,241:00)<br>(C07D487/04, 253:00,249:00) |
| D,Y | EP-A-0 369 743 (IMPERIAL CHEMICAL INDUSTRIES)<br>* Seite 2, Zeile 1 - Seite 2, Zeile 24; Ansprüche 1,9 * | 1,24,26 | |
| P,X | EP-A-0 464 572 (F. HOFFMANN-LA ROCHE)<br>* Ansprüche 1,22,24 * | 1,24,26 | |

RECHERCHIERTE SACHGEBIETE (Int. Cl.5 )

C07D
A61K

Der vorliegende Recherchenbericht wurde für alle Patentansprüche erstellt

| Recherchenort | Abschlußdatum der Recherche | Prüfer |
|---|---|---|
| DEN HAAG | 22 FEBRUAR 1993 | VOYIAZOGLOU D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument